# EUROPEAN PATENT SPECIFICATION

(11) **EP 2 179 057 B1**
(45) Date of publication and mention of the grant of the patent: **03.10.2012**
(21) Application number: 08785427.9
(22) Date of filing: 07.08.2008
(51) Int. Cl.: C12Q 1/68

(54) **PREDICTIVE MARKER FOR EGFR INHIBITOR TREATMENT**
PROGNOSEMARKER ZUR BEHANDLUNG MIT EGFR-HEMMERN
MARQUEUR PRÉDICTIF POUR UN TRAITEMENT PAR INHIBITEUR D'EGFR

(30) Priority: 14.08.2007 EP 07114309
(43) Date of publication of application: 28.04.2010
(73) Proprietor: F. Hoffmann-La Roche AG, 4070 Basel (CH)
(72) Inventor: DELMAR, Paul, CH-4057 Basel (CH); KLUGHAMMER, Barbara, 79618 Rheinfelden (DE); LUTZ, Verena, 81371 Muenchen (DE); MCLOUGHLIN, Patricia, CH-4057 BASEL (CH)
(74) Representative: Halbig, Dirk
(86) International application number: PCT/EP2008/006521
(87) International publication number: WO 2009/021682

(56) References cited:
- WO-A-2004/079012
- WO-A-2004/111273
- WO-A-2005/047451
- WO-A-2005/049829
- KAKIUCHI SOJI ET AL: "Prediction of sensitivity of advanced non-small cell lung cancers to gefitinib (Iressa, ZD1839)" HUMAN MOLECULAR GENETICS, OXFORD UNIVERSITY PRESS, SURREY, vol. 13, no. 24, 15 December 2004 (2004-12-15), pages 3029-3043, XP002440000 ISSN: 0964-6906
- OKANO TETSUYA ET AL: "Proteomic signature corresponding to the response to gefitinib (Iressa, ZD1839), an epidermal growth factor receptor tyrosine kinase inhibitor in lung adenocarcinoma" CLINICAL CANCER RESEARCH, THE AMERICAN ASSOCIATION FOR CANCER RESEARCH, US, vol. 13, no. 3, 1 February 2007 (2007-02-01), pages 799-805, XP002440001 ISSN: 1078-0432
- KOKUBO Y ET AL: "Reduction of PTEN protein and loss of epidermal growth factor receptor gene mutation in lung cancer with natural resistance to gefitinib (IRESSA)" BRITISH JOURNAL OF CANCER, NATURE PUBLISHING GROUP, LONDON, GB, vol. 92, 1 January 2005 (2005-01-01), pages 1711-1719, XP007905848 ISSN: 0007-0920
- ZHOU BIN-BING S ET AL: "Targeting ADAM-mediated ligand cleavage to inhibit HER3 and EGFR pathways in non-small cell lung cancer" CANCER CELL, CELL PRESS, US, vol. 10, no. 1, 1 January 2006 (2006-01-01), pages 39-50, XP008093928 ISSN: 1535-6108
- CESARE GRIDELLI ET AL: "Erlotinib in Non-Small Cell Lung Cancer Treatment: Current Status and Future Development" ONCOLOGIST, ALPHAMED PRESS, US, vol. 12, 1 January 2007 (2007-01-01), pages 840-849, XP007905850 ISSN: 1083-7159
- ACHIWA HIROYUKI ET AL: "PRL-1 tyrosine phosphatase regulates c-Src levels, adherence, and invasion in human lung cancer cells" CANCER RESEARCH, vol. 67, no. 2, January 2007 (2007-01), pages 643-650, XP002501112 ISSN: 0008-5472

## Description

The present invention provides a biomarker that is predictive for the clinical benefit of EGFR inhibitor treatment in cancer patients.

A number of human malignancies are associated with aberrant or over-expression of the epidermal growth factor receptor (EGFR). EGF, transforming growth factor-α (TGF-α), and a number of other ligands bind to the EGFR, stimulating autophosphorylation of the intracellular tyrosine kinase domain of the receptor. A variety of intracellular pathways are subsequently activated, and these downstream events result in tumour cell proliferation in vitro. It has been postulated that stimulation of tumour cells via the EGFR may be important for both tumour growth and tumour survival in vivo.

Early clinical data with Tarceva™, an inhibitor of the EGFR tyrosine kinase, indicate that the compound is safe and generally well tolerated at doses that provide the targeted effective concentration (as determined by preclinical data). Clinical phase I and II trials in patients with advanced disease have demonstrated that Tarceva™ has promising clinical activity in a range of epithelial tumours. Indeed, Tarceva™ has been shown to be capable of inducing durable partial remissions in previously treated patients with head and neck cancer, and NSCLC (Non small cell lung cancer) of a similar order to established second line chemotherapy, but with the added benefit of a better safety profile than chemo therapy and improved convenience (tablet instead of intravenous [i.v.] administration). A recently completed, randomised, double-blind, placebo-controlled trial (BR.21) has shown that single agent Tarceva™ significantly prolongs and improves the survival of NSCLC patients for whom standard therapy for advanced disease has failed.

Tarceva™ (erlotinib) is a small chemical molecule; it is an orally active, potent, selective inhibitor of the EGFR tyrosine kinase (EGFR-TKI).

Lung cancer is the major cause of cancer-related death in North America and Europe. In the United States, the number of deaths secondary to lung cancer exceeds the combined total deaths from the second (colon), third (breast), and fourth (prostate) leading causes of cancer deaths combined. About 75% to 80% of all lung cancers are NSCLC, with approximately 40% of patients presenting with locally advanced and/or unresectable disease. This group typically includes those with bulky stage IIIA and IIIB disease, excluding malignant pleural effusions.

The crude incidence of lung cancer in the European Union is 52.5, the death rate 48.7 cases/100000/year. Among men the rates are 79.3 and 78.3, among women 21.6 and 20.5, respectively. NSCLC accounts for 80% of all lung cancer cases. About 90% of lung cancer mortality among men, and 80% among women, is attributable to smoking.

In the US, according to the American Cancer Society, during 2004, there were approximately 173,800 new cases of lung cancer (93,100 in men and 80,700 in women) and were accounting for about 13% of all new cancers. Most patients die as a consequence of their disease within two years of diagnosis. For many NSCLC patients, successful treatment remains elusive. Advanced tumours often are not amenable to surgery and may also be resistant to tolerable doses of radiotherapy and chemotherapy. In randomized trials the currently most active combination chemotherapies achieved response rates of approximately 30% to 40% and a 1-year survival rate between 35% and 40%. This is really an advance over the 10% 1-year survival rate seen with supportive care alone (Shepherd 1999).

Until recently therapeutic options for relapsed patients following relapse were limited to best supportive care or palliation. A recent trial comparing docetaxel (Taxotere) with best supportive care showed that patients with NSCLC could benefit from second line chemotherapy after cisplatin-based first-line regimens had failed. Patients of all ages and with ECOG performance status of 0, 1, or 2 demonstrated improved survival with docetaxel, as did those who had been refractory to prior platinum-based treatment. Patients who did not benefit from therapy included those with weight loss of 10%, high lactate dehydrogenase levels, multi-organ involvement, or liver involvement. Additionally, the benefit of docetaxel monotherapy did not extend beyond the second line setting. Patients receiving docetaxel as third-line treatment or beyond showed no prolongation of survival. Single-agent docetaxel became a standard second-line therapy for NSCLC. Recently another randomized phase III trial in second line therapy of NSCLC compared pemetrexed (Alimta®) with docetaxel. Treatment with pemetrexed resulted in a clinically equivalent efficacy but with significantly fewer side effects compared with docetaxel.

It has long been acknowledged that there is a need to develop methods of individualising cancer treatment. With the development of targeted cancer treatments, there is a particular interest in methodologies which could provide a molecular profile of the tumour target, (i.e. those that are predictive for clinical benefit). Proof of principle for gene expression profiling in cancer has already been established with the molecular classification of tumour types which are not apparent on the basis of current morphological and immunohistochemical tests. Two separate disease entities were differentiated with differing prognoses from the single current classification of diffuse large B-cell lymphoma using gene expression profiling.

Therefore, it is an aim of the present invention to provide expression biomarkers that are predictive for the clinical benefit of EGFR inhibitor treatment in cancer patients.
The following documents all disclose the prediction of the response of a NSCLC to treatment with an EGFR inhibitor, mostly based on expression profiling:
WO 2005/049829;
WO 2004/111273;
KAKIUCHI SOJI ET AL: "Prediction of sensitivity of advanced non-small cell lung cancers to gefitinib (Iressa, ZD1839)", HUMAN MOLECULAR GENETICS, vol. 13, no. 24, 15 December 2004, pages 3029-3043;
OKANO TETSUYA ET AL: "Proteomic signature corresponding to the response to gefitinib (Iressa, ZD1839), an epidermal growth factor receptor tyrosine kinase inhibitor in lung adenocarcinoma", CLINICAL CANCER RESEARCH, vol. 13, no. 3, pages 799-805.

In a first object the present invention provides an in vitro method of predicting the clinical benefit of a cancer patient in response to treatment with an EGFR inhibitor comprising the steps: determining an expression level of a PTP4A1 gene in a tumour sample of a patient and comparing the expression level of the PTP4A1 gene to a value representative of an expression level of the PTP4A1 gene in tumours of a population of patients deriving no clinical benefit from the treatment, wherein a lower expression level of the PTP4A1 gene in the tumour sample of the patient is indicative for a patient who will derive clinical benefit from the treatment.

The abbreviation PTP4A1 means protein tyrosine phosphatase type IVA, member 1. Seq. Id. No. 1 shows the nucleotide sequence of human PTP4A1.

The term "a value representative of the expression level of the PTP4A1 gene in tumours of a population of patients deriving no clinical benefit from the treatment" refers to an estimate of the mean expression level of the marker gene in tumours of a population of patients who do not derive a clinical benefit from the treatment. Clinical benefit was defined as either having an objective response or disease stabilization for ≥12 weeks.

In a further preferred embodiment, the PTP4A1 gene shows between 1.2 and 1.8 or more fold lower expression level in the tumour sample of the patient compared to a value representative of the population of patients deriving no clinical benefit from the treatment.

In a preferred embodiment, the expression level of the marker gene is determined by microarray technology or other technologies that assess RNA expression levels like quantitative RT-PCR, or by any method looking at the expression level of the respective protein, eg immunohistochemistry (IHC). The construction and use of gene chips are well known in the art. see, U. S. Pat Nos. 5,202,231; 5,445,934; 5,525,464; 5,695,940; 5,744,305; 5,795, 716 and 1 5,800,992. See also, Johnston, M. Curr. Biol. 8:R171-174 (1998); Iyer VR et al., Science 283:83-87 (1999). Of course, the gene expression level can be determined by other methods that are known to a person skilled in the art such as e.g. northern blots, RT-PCR, real time quantitative PCR, primer extension, RNase protection, RNA expression profiling.

The marker gene of the present invention can be combined with other biomarkers to biomarker sets. Biomarker sets can be built from any combination of predictive biomarkers to make predictions about the effect of EGFR inhibitor treatment in cancer patients. The biomarkers and biomarkers sets described herein can be used, for example, to predict how patients with cancer will respond to therapeutic intervention with an EGFR inhibitor.

The term "gene" as used herein comprises variants of the gene. The term "variant" relates to nucleic acid sequences which are substantially similar to the nucleic acid sequences given by the GenBank accession number. The term "substantially similar" is well understood by a person skilled in the art. In particular, a gene variant may be an allele which shows nucleotide exchanges compared to the nucleic acid sequence of the most prevalent allele in the human population. Preferably, such a substantially similar nucleic acid sequence has a sequence similarity to the most prevalent allele of at least 80%, preferably at least 85%, more preferably at least 90%, most preferably at least 95%. The term "variants" is also meant to relate to splice variants.

The EGFR inhibitor can be selected from the group consisting of gefitinib, erlotinib, PKI-166, EKB-569, GW2016, CI-1033 and an anti-erbB antibody such as trastuzumab and cetuximab.

In another embodiment, the EGFR inhibitor is erlotinib.

In yet another embodiment, the cancer is NSCLC.

Techniques for the detection and quantification of gene expression of the genes described by this invention include, but are not limited to northern blots, RT-PCR, real time quantitative PCR, primer extension, RNase protection, RNA expression profiling and related techniques. These techniques are well known to those of skill in the art see e.g. Sambrook J et al., Molecular Cloning: A Laboratory Manual, Third Edition (Cold Spring Harbor Press, Cold Spring Harbor, 2000).

Techniques for the detection of protein expression of the respective genes described by this invention include, but are not limited to immunohistochemistry (IHC).

In accordance with the invention, cells from a patient tissue sample, e.g., a tumour or cancer biopsy, can be assayed to determine the expression pattern of one or more biomarkers. Success or failure of a cancer treatment can be determined based on the biomarker expression pattern of the cells from the test tissue (test cells), e.g., tumour or cancer biopsy, as being relatively similar or different from the expression pattern of a control set of the one or more biomarkers. In the context of this invention, it was found that the gene of table 3 is down regulated i.e. shows a lower expression level, in tumours of patients who derived clinical benefit from EGFR inhibitor treatment compared to tumours of patients who did not derive clinical benefit from the EGFR inhibitor treatment. Thus, if the test cells show a biomarker expression profile which corresponds to that of a patient who responded to cancer treatment, it is highly likely or predicted that the individual's cancer or tumour will respond favorably to treatment with the EGFR inhibitor. By contrast, if the test cells show a biomarker expression pattern corresponding to that of a patient who did not respond to cancer treatment, it is highly likely or predicted that the individual's cancer or tumour will not respond to treatment with the EGFR inhibitor.

The biomarker of the present invention i.e. the gene listed in table 3, is a first step towards an individualized therapy for patients with cancer, in particular patients with refractory NSCLC. This individualized therapy will allow treating physicians to select the most appropriate agent out of the existing drugs for cancer therapy, in particular NSCLC. The benefit of individualized therapy for each future patient are: response rates / number of benefiting patients will increase and the risk of adverse side effects due to ineffective treatment will be reduced.

In a further object the present invention provides a therapeutic method of treating a cancer patient identified by the in vitro method of the present invention. Said therapeutic method comprises administering an EGFR inhibitor to the patient who has been selected for treatment based on the predictive expression pattern of the gene of table 3. A preferred EGFR inhibitor is erlotinib and a preferred cancer to be treated is NSCLC.

### Short description of the figures

Figure 1 shows the study design;
Figure 2 shows the scheme of sample processing;
Figure 3a shows PTP4A1 expression levels versus clinical outcome for Genechip® profiling;
Figure 3b shows PTP4A1 expression levels versus clinical outcome for qRT-PCR and
Figure 3c shows the correlation between Genechip® and qRT-PCR measurements for PTP4A1.

### Experimental part

### Rationale for the Study and Study Design

Recently mutations within the EGFR gene in the tumour tissue of a subset of NSCLC patients and the association of these mutations with sensitivity to erlotinib and gefitinib were described (Pao W, et al. 2004; Lynch et al. 2004; Paez et al. 2004). For the patients combined from two studies, mutated EGFR was observed in 13 of 14 patients who responded to gefitinib and in none of the 11 gefitinib-treated patients who did not respond. The reported prevalence of these mutations was 8% (2 of 25) in unselected NSCLC patients. These mutations were found more frequently in adenocarcinomas (21 %), in tumours from females (20%), and in tumours from Japanese patients (26%). These mutations result in increased in vitro activity of EGFR and increased sensitivity to gefitinib. The relationship of the mutations to prolonged stable disease or survival duration has not been prospectively evaluated.

Based on exploratory analyses from the BR.21 study, it appeared unlikely that the observed survival benefit is only due to the EGFR mutations, since a significant survival benefit is maintained even when patients with objective response are excluded from analyses (data on file). Other molecular mechanisms must also contribute to the effect.

Based on the assumption that there are changes in gene expression levels that are predictive of response / benefit to Tarceva™ treatment, microarray analysis was used to detect these changes

This required a clearly defined study population treated with Tarceva™ monotherapy after failure of 1st line therapy. Based on the experience from the BR.21 study, benefiting population was defined as either having objective response, or disease stabilization for 12 weeks. Clinical and microarray datasets were analyzed according to a pre-defined statistical plan.

The application of this technique requires fresh frozen tissue (FFT). Therefore a mandatory biopsy had to be performed before start of treatment. The collected material was frozen in liquid nitrogen (N2).

A second tumour sample was collected at the same time and stored in paraffin (formalin fixed paraffin embedded, FFPE). This sample was analysed for alterations in the EGFR signaling pathway.

The ability to perform tumour biopsies via bronchoscopy was a prerequisite for this study. Bronchoscopy is a standard procedure to confirm the diagnosis of lung cancer. Although generally safe, there is a remaining risk of complications, e.g. bleeding.

This study was a first step towards an individualized therapy for patients with refractory NSCLC. This individualized therapy will allow treating physicians to select the most appropriate agent out of the existing drugs for this indication.

Once individualized therapy will be available, the benefit for each future patient will outweigh the risk patients have to take in the present study:
response rates / number of benefiting patients will increase,
the risk of adverse side effects due to ineffective treatment will be reduced.

### Rationale for Dosage Selection

Tarceva™ was given orally once per day at a dose of 150 mg until disease progression, intolerable toxicities or death. The selection of this dose was based on pharmacokinetic parameters, as well as the safety and tolerability profile of this dose observed in Phase I, II and III trials in heavily pre-treated patients with advanced cancer. Drug levels seen in the plasma of patients with cancer receiving the 150 mg/day dose were consistently above the average plasma concentration of 500 ng / ml targeted for clinical efficacy. BR.21 showed a survival benefit with this dose.

### Objectives of the Study

The primary objective was the identification of differentially expressed genes that are predictive for benefit (CR, PR or SD ? 12 weeks) of Tarceva™ treatment. Identification of differentially expressed genes predictive for "response" (CR, PR) to Tarceva™ treatment was an important additional objective.

The secondary objectives were to assess alterations in the EGFR signaling pathways with respect to benefit from treatment.

### Study Design

### Overview of Study Design and Dosing Regimen

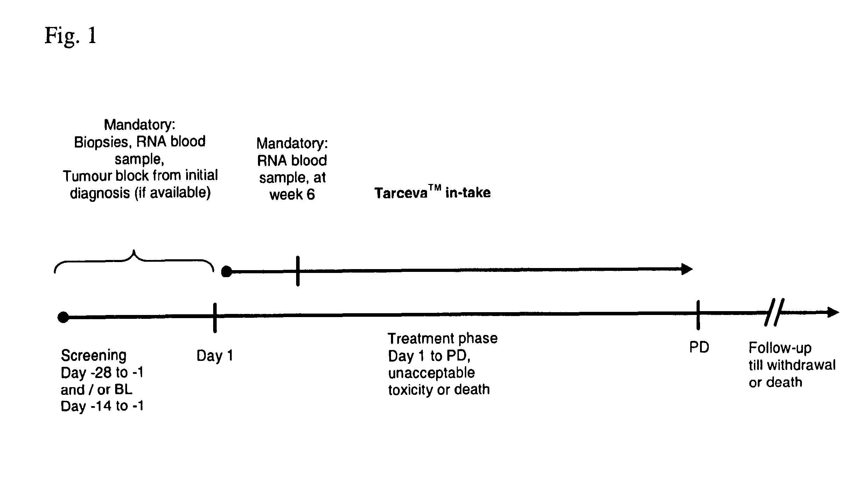

This was an open-label, predictive marker identification Phase II study. The study was conducted in approximately 26 sites in about 12 countries. 264 patients with advanced NSCLC following failure of at least one prior chemotherapy regimen were enrolled over a 12 month period. Continuous oral Tarceva™ was given at a dose of 150 mg/day. Dose reductions were permitted based on tolerability to drug therapy. Clinical and laboratory parameters were assessed to evaluate disease control and toxicity. Treatment continued until disease progression, unacceptable toxicity or death. The study design is depicted in figure 1.

Tumour tissue and blood samples were obtained for molecular analyses to evaluate the effects of Tarceva™ and to identify subgroups of patients benefiting from therapy.

### Predictive Marker Assessments

Biopsies of the tumour were taken within 2 weeks before start of treatment. Two different samples were collected:
The first sample was always frozen immediately in liquid N2
The second sample was fixed in formalin and embedded in paraffin
Snap frozen tissue had the highest priority in this study.

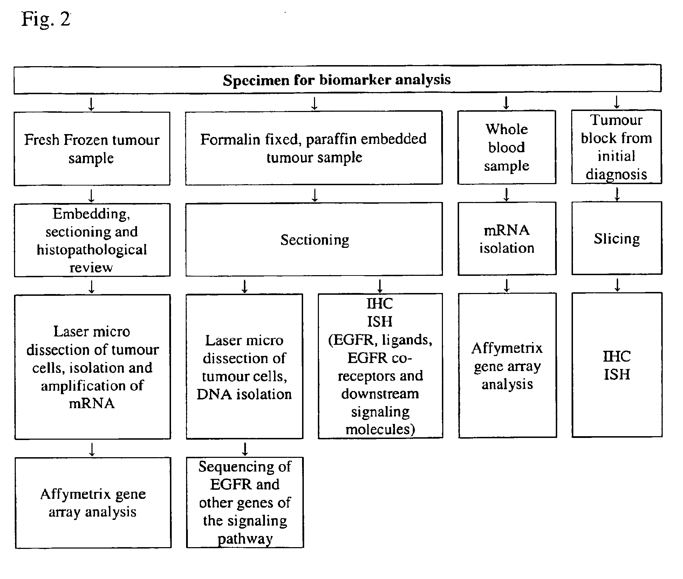

Figure 2 shows a scheme of the sample processing.

### Microarray Analysis

The snap frozen samples were used for laser capture microdissection (LCM) of tumour cells to extract tumour RNA and RNA from tumour surrounding tissue. The RNA was analysed on Affymetrix microarray chips (HG-U133A) to establish the patients' tumour gene expression profile. Quality Control of Affymetrix chips was used to select those samples of adequate quality for statistical comparison.

### Single Biomarker Analyses on Formalin Fixed Paraffin Embedded Tissue

The second tumour biopsy, the FFPE sample, was used to perform DNA mutation, IHC and ISH analyses as described below. Similar analyses were performed on tissue collected at initial diagnosis.

The DNA mutation status of the genes encoding EGFR and other molecules involved in the EGFR signaling pathway were analysed by DNA sequencing. Gene amplification of EGFR and related genes were be studied by FISH.

Protein expression analyses included immunohistochemical [IHC] analyses of EGFR and other proteins within the EGFR signalling pathway.

### Response Assessments

The RECIST (Uni-dimensional Tumour Measurement) criteria were used to evaluate response. These criteria can be found under the following link: http://www.eortc.be/recist/

Note that:To be assigned a status of CR or PR, changes in tumour measurements must be confirmed by repeated assessments at least 4 weeks apart at any time during the treatment period.

In the case of SD, follow-up measurements must have met the SD criteria at least once after study entry at a minimum interval of 6 weeks.

In the case of maintained SD, follow-up measurements must have met the SD criteria at least once after study entry with maintenance duration of at least 12 weeks.

### Survival Assessment

A regular status check every 3 months was performed either by a patient's visit to the clinic or by telephone. All deaths were recorded. At the end of the study a definitive confirmation of survival was required for each patient.

### Methods

### RNA sample preparation and quality control of RNA samples

All biopsy sample processing was handled by a pathology reference laboratory; fresh frozen tissue samples were shipped from investigator sites to the Clinical Sample Operations facility in Roche Basel and from there to the pathology laboratory for further processing. Laser capture microdissection was used to select tumour cells from surrounding tissue. After LCM, RNA was purified from the enriched tumour material. The pathology laboratory then carried out a number of steps to make an estimate of the concentration and quality of the RNA.

RNases are RNA degrading enzymes and are found everywhere and so all procedures where RNA will be used must be strictly controlled to minimize RNA degradation. Most mRNA species themselves have rather short half-lives and so are considered quite unstable. Therefore it is important to perform RNA integrity checks and quantification before any assay.

RNA concentration and quality profile can be assessed using an instrument from Agilent (Agilent Technologies, Inc., Palo Alto, CA) called a 2100 Bioanalyzer®. The instrument software generates an RNA Integrity Number (RIN), a quantitation estimate (Schroeder, A., et al., The RIN: an RNA integrity number for assigning integrity values to RNA measurements. BMC Mol Biol, 2006. 7: p. 3), and calculates ribosomal ratios of the total RNA sample. The RIN is determined from the entire electrophoretic trace of the RNA sample, and so includes the presence or absence of degradation products.

The RNA quality was analysed by a 2100 Bioanalyzer®. Only samples with at least one rRNA peak above the added poly-I noise and sufficient RNA were selected for further analysis on the Affymetrix platform. The purified RNA was forwarded to the Roche Centre for Medical Genomics (RCMG; Basel, Switzerland) for analysis by microarray. 122 RNA samples were received from the pathology lab for further processing.

### Target Labeling of tissue RNA samples

Target labeling was carried out according to the Two-Cycle Target Labeling Amplification Protocol from Affymetrix (Affymetrix, Santa Clara, California), as per the manufacturer's instructions.

The method is based on the standard Eberwine linear amplification procedure but uses two cycles of this procedure to generate sufficient labeled cRNA for hybridization to a microarray.

Total RNA input used in the labeling reaction was 10ng for those samples where more than 10ng RNA was available; if less than this amount was available or if there was no quantity data available (due to very low RNA concentration), half of the total sample was used in the reaction. Yields from the labeling reactions ranged from 20-180µg cRNA. A normalization step was introduced at the level of hybridization where 15 µg cRNA was used for every sample.

Human Reference RNA (Stratagene, Carlsbad, CA, USA) was used as a control sample in the workflow with each batch of samples. 10ng of this RNA was used as input alongside the test samples to verify that the labeling and hybridization reagents were working as expected.

### Microarray hybridizations

Affymetrix HG-U133A microarrays contain over 22,000 probe sets targeting approximately 18,400 transcripts and variants which represent about 14,500 well-characterized genes.

Hybridization for all samples was carried out according to Affymetrix instructions (Affymetrix Inc., Expression Analysis Technical Manual, 2004). Briefly, for each sample, 15µg of biotin-labeled cRNA were fragmented in the presence of divalent cations and heat and hybridized overnight to Affymetrix HG-U133A full genome oligonucleotide arrays. The following day arrays were stained with streptavidin-phycoerythrin (Molecular Probes; Eugene, OR) according to the manufacturer's instructions. Arrays were then scanned using a GeneChip Scanner 3000 (Affymetrix), and signal intensities were automatically calculated by GeneChip Operating Software (GCOS) Version 1.4 (Affymetrix).

### Statistical Analysis

Analysis of the Affymetrix™ data consisted of five main steps.

Step 1 was quality control. The goal was to identify and exclude from analysis array data with a sub-standard quality profile.

Step 2 was pre-processing and normalization. The goal was to create a normalized and scaled "analysis data set", amenable to inter-chip comparison. It comprised background noise estimation and subtraction, probe summarization and scaling.

Step 3 was exploration and description. The goal was to identify potential bias and sources of variability. It consisted of applying multivariate and univariate descriptive analysis techniques to identify influential covariates.

Step 4 was modeling and testing. The goal was to identify a list of candidate markers based on statistical evaluation of the difference in mean expression level between "clinical benefit" and "no clinical benefit" patients. It consisted in fitting an adequate statistical model to each probe-set and deriving a measure of statistical significance.

Step 5 was a robustness analysis. The goal was to generate a qualified list of candidate markers that do not heavily depend on the pre-processing methods and statistical assumptions. It consisted in reiterating the analysis with different methodological approaches and intersecting the list of candidates. All analyses were performed using the R software package.

### Step 1: Quality Control

The assessment of data quality was based on checking several parameters. These included standard Affymetrix GeneChip™ quality parameters, in particular: Scaling Factor, Percentage of Present Call and Average Background. This step also included visual inspection of virtual chip images for detecting localized hybridization problems, and comparison of each chip to a virtual median chip for detecting any unusual departure from median behaviour. Inter-chip correlation analysis was also performed to detect outlier samples. In addition, ancillary measures of RNA quality obtained from analysis of RNA samples with the Agilent Bioanalyzer™ 2100 were taken into consideration.

Based on these parameters, data from 20 arrays were excluded from analysis. Thus data from a total of 102 arrays representing 102 patients was included in the analysis. The clinical description of these 102 samples set is reported in table 1.

**Table 1: Description of clinical characteristics of patients included in the analysis**

| **Variable** | **Value** | **n=102** |
|---|---|---|
| | | n (%) |
| Best Response | N/A | 16 (15.7%) |
| | PD | 49 (48.0%) |
| | SD | 31 (30.4%) |
| | PR | 6 (5.9%) |
| | | |
| Clinical Benefit | NO | 81 (79.4%) |
| | YES | 21 (20.6%) |
| | | |
| SEX | FEMALE | 25 (24.5%) |
| | MALE | 77 (74.5%) |
| | | |
| ETHNICITY | CAUCASIAN | 65 (63.7%) |
| | ORIENTAL | 37 (36.3%) |
| | | |
| Histology | ADENOCARCINOMA | 35 (34.3%) |
| | SQUAMOUS | 53 (52.0%) |
| | OTHERS | 14 (13.7%) |
| | | |
| Ever-Smoking | NO | 20 (19.6%) |
| | YES | 82 (80.4%) |

### Step 2: Data pre-processing and normalization

The rma algorithm (Irizarry, R.A., et al., Summaries of Affymetrix GeneChip probe level data. Nucl. Acids Res., 2003. 31(4): p. e15) was used for pre-processing and normalization. The mas5 algorithm (AFFYMETRIX, GeneChip® Expression: Data Analysis Fundamentals. 2004, AFFYMETRIX) was used to make detection calls for the individual probe-sets. Probe-sets called "absent" or "marginal" in all samples were removed from further analysis; 5930 probe-sets were removed from analysis based on this criterion. The analysis data set therefore consisted of a matrix with 16353 (out of 22283) probe-sets measured in 102 patients.

### Step 3: Data description and exploration

Descriptive exploratory analysis was performed to identify potential bias and major sources of variability. A set of covariates with a potential impact on gene expression profiles was screened. It comprised both technical and clinical variables. Technical covariates included: date of RNA processing (later referred to as batch), RIN (as a measure of RNA quality/integrity), Operator and Center of sample collection. Clinical covariates included: Histology type, smoking status, tumour grade, performance score (Oken, M.M., et al., Toxicity and response criteria of the Eastern Cooperative Oncology Group. Am J Clin Oncol, 1982. 5(6): p. 649-55), demographic data, responder status and clinical benefit status.

The analysis tools included univariate ANOVA and principal component analysis. For each of these covariates, univariate ANOVA was applied independently to each probe-set.

A significant effect of the batch variable was identified. In practice, the batch variable captured differences between dates of sample processing and Affymetrix chip lot. After checking that the batch variable was nearly independent from the variables of interest, the batch effect was corrected using the method described in Johnson, W.E., C. Li, and A. Rabinovic, Adjusting batch effects in microarray expression data using empirical Bayes methods. Biostat, 2007. 8(1): p. 118-127.

The normalized data set after batch effect correction served as the analysis data set in subsequent analyses.

Histology and RIN were two additional important variables highlighted by the descriptive analysis.

### Step 4: Data modeling and testing.

A linear model was fitted independently to each probe-set. Variables included in the model are reported in table 2. The model parameters were estimated by the maximum likelihood technique. The parameter corresponding to the "Clinical Benefit" variable (X1) was used to assess the difference in expression level between the group of patients with clinical benefit and the group with no clinical benefit.

**Table 2: Description of the variables included in the linear model.**

| **Variable** | **Type** | **Value** |
|---|---|---|
| **gene expression** | **Dependent (Yᵢₚ)** | **log2 intensity of probe-set i in patient p.** |
| **Intercept** | **Overall mean (µ)** | |
| **Clinical Benefit** | **Predictor of interest (X1)** | **YES / NO** |
| **Histology** | **Adjustment Covariate (X2)** | **ADENO. / SQUAM. / OTHERS** |
| **RACE** | **Adj. Cov. (X3)** | **ORIENT. / CAUCAS.** |
| **SEX** | **Adj. Cov. (X4)** | **FEMALE / MALE** |
| **RIN** | **Adj. Cov. (X5)** | **[2,...,7.9]** |
| **SMOKER** | **Adj. Cov. (X6)** | **CURRENT/PAST/NEVER** |
| **Stage** | **Adj. Cov. (X7)** | **UNRESECT.III / IV** |

For each probe-set i, the aim of the statistical test was to reject the hypothesis that the mean expression levels in patients with clinical benefit and patients without clinical benefit are equal, taking into account the other adjustment covariates listed in table 2. Formally, the null hypothesis of equality was tested against a two sided alternative. The corresponding p-values are reported in table 3.

The choice of linear model was motivated by two reasons. Firstly, linear modeling is a versatile, well-characterized and robust approach that allows for adjustment of confounding variables when estimating the effect of the variable of interest. Secondly, given the sample size of 102, and the normalization and scaling of the data set, the normal distribution assumption was reasonable and justified.

For each probe-set, the assumption of homogeneity of variance was evaluated using Fligner-Killeen tests based on the model residuals. The analysis consisted of 3 steps :
1. Test each categorical variables for homogeneity of residual variance
2. Note the variable V with the least p-value
3. If the least p-value is less than 0.001, re-fit the model allowing the different level of variables V to have a different variance.

### Step 5: Robustness

The goal of the robustness analysis was to reduce the risk that the results of the analysis might be artifactual and a result of the pre-processing steps or assumptions underlying the statistical analysis. The following three aspects were considered: a) inclusion or exclusion of a few extra chips at the quality control step; b) pre-processing and normalization algorithm; c) statistical assumptions and testing approach.

The list of candidate markers was defined as the subset of genes consistently declared as significant with different analysis settings. The different applied analysis options were the following:
a) An additional subset of 8 chips was identified based on more stringent quality control criteria. A "reduced data set" was defined by excluding these 8 chips.
b) MAS5 was identified as an alternative to rma for pre-processing and normalization. MAS5 uses different methods for background estimation, probe summarization and normalization.
c) Two additional statistical tests were employed.

a. A wilcoxon test for the difference between clinical and no clinical benefit and
b. a likelihood ratio test (LRT) testing for the logistic regression model where clinical benefit was taken as the response variable and gene expression as covariate. These two additional tests rely on a different set of underlying statistical assumptions. For each probe-set, the LRT was following a Chi-square with 1 degree of freedom.

In summary, two sets of samples (the "full" data-set and the "reduced" data-set), and 2 pre-processing algorithm (mas5 and rma) were considered; this resulted in four different analysis data sets. To each of these four data sets, three different statistical tests were applied. Therefore, for each probe-set, three p-values were calculated. In each analysis data set, a composite criterion was applied to identify the list of differentially regulated genes. This composite criterion was defined as: the maximum p-value is less than 0.05 and the minimum p-values is less than 0.001. The robustness analysis using criterion 1 for identifying marker genes yielded PTP4A1 as predictive marker for EGFR inhibitor treatment.

**Table 3:**

| | | | | | |
|---|---|---|---|---|---|
| Gene marker for Clinical Benefit based on the robustness analysis after application of the composite Criterion. Column 1 is the Affymetrix identifier of the probe-set. Column 2 is the GenBank accession number of the corresponding gene sequence. Column 3 is the corresponding official gene name. Column 4 is the corresponding adjusted mean fold change in expression level between clinical and no clinical benefit patient, as estimated from the linear model. Column 5 is the p-value for the test of difference in expression level between clinical benefit and no clinical benefit patients as derived from the linear model. Column 6 is the 95% confidence interval for the adjusted mean fold change in expression level. | | | | | |

| **Affymetrix Probe Set ID** | **GenBank** | **Gene** | **Adjusted Mean Fold Change** | **P-value** | **CI 95 %** |
|---|---|---|---|---|---|
| 200732_s_at | NM_003463 (Seq. Id. No. 1) | PTP4A1 | -1.44 | 3.2.1E-3 | -1.8 , -1.2 |

### Further statistical analysis

For the selected candidate marker PTP4A1, the following additional analyses were performed in a validated environment by an independent statisticians :
- Univariate Cox Regression for PFS (Progression free survival) from Primary Affymetrix Analysis,
- Univariate Logistic Regression for Clinical Benefit from Primary Affymetrix Analysis, and
- Univariate Cox Regression for Survival from Primary Affymetrix Analysis

The results of these analysis are presented below. They are consistent with the results of the primary analysis and confirm the choice of the selected marker.

Results: Univariate Cox Regression for PFS (Progression free survival) from Primary Affymetrix Analysis:

| **Gene** | **No. of patients** | **Hazard ratio** | **95 % CI for Hazard ratio** | **p-Value** |
|---|---|---|---|---|
| PTP4A1 | 102 | 1.69 | 1.23; 2.33 | 0.0011 |

Results: Univariate Cox Regression for Clinical benefit from Primary Affymetrix Analysis:

| **Gene** | **No. of patients** | **Odds ratio** | **95 % CI for Odds ratio** | **p-Value** |
|---|---|---|---|---|
| PTP4A1 | 102 | 0.22 | 0.08; 0.57 | 0.0018 |

Results: Univariate Cox Regression for Survival from Primary Affymetrix Analysis:

| **Gene** | **No. of patients** | **Hazard ratio** | **95 % CI for Hazard ratio** | **p-Value** |
|---|---|---|---|---|
| PTP4A1 | 102 | 1.63 | 1.15; 2.30 | 0.0055 |

### qRT-PCR

cDNA was synthesized using SuperScriptTM III First-strand Synthesis SuperMix for qRT-PCR (Invitrogen, CA, USA) according to the manufacturer's instructions but without inclusion of an RNase H digest.

Quantitative PCR was performed using TaqMan® Gene Expression Assays on an ABI PRISM@ 7900HT Sequence Detection System according to the manufacturer's recommendations (Applied Biosystems, CA, USA). All assays were performed in triplicate.

The used primers and probes crossed exon boundaries or were within the Affymetrix Genechip® probe sequence of interest. Two house-keeping genes were included as endogenous controls: beta-2-microglobulin (B2M; Assay Hs99999907_ml) and hypoxanthinephosphoribosyl transferase (HPRT; Assay Hs99999909_ml).

All runs included a calibrator sample (MVPTM total RNA from human adult lung; Stratagene, CA, USA) and a standard curve. Universal Human Reference total RNA (Stratagene, CA, USA) was used as template for PTPRF standard curves. All samples were measured in triplicate. Relative quantification was performed using the -ΔCt method.

### Results

As reported previously, Affymetrix Genechip® gene expression profiles were determined for 102 patients included in this study. Among these patients, qRT-PCR results were obtained for 75 (table 4). The demographics and clinical characteristics of the patients with qRT-PCR results were similar to those of the entire population (n=264) and of the patients with Genechip® gene expression profiles available.

**Table 4: Baseline characteristics: patients with qRT-PCR analyses (n=75)**

| **Characteristic** | | |
|---|---|---|
| Age (median, range) | | 62 (39-85) |
| Gender; n (%) | | |
| | Male | 19 (25) |
| | Female | 56 (75) |
| ECOG performance status; n (%) | | |
| | 0 | 7 (9) |
| | 1 | 45 (60) |
| | 2 | 23 (31) |
| Histology; n (%) | | |
| | Adenocarcinoma | 27 (36) |
| | Squamous-cell carcinoma | 34 (45) |
| | Large-cell carcinoma | 2 (3) |
| | Other | 12 (16) |
| Disease stage; n (%) | | |
| | IIIB | 22 (29) |
| | IV | 53 (71) |
| Number of prior chemotherapy regimens; n (%) | | |
| | 0 | 19 (25) |
| | 1 | 36 (48) |
| | ≥2 | 20 (27) |
| Ethnicity; n (%) | | |
| | Caucasian | 51 (68) |
| | Asian | 24 (32) |
| Smoking history; n (%) | | |
| | Never | 12 (16) |
| | Current | 24 (32) |
| | Former | 39 (52) |

Of the 75 patients with qRT-PCR results, 4 (5%) had partial response (PR), 23 (31%) had SD, 39 (52%) had PD, and 9 (12%) were not evaluable. These results were very similar to those observed in the entire study population (n=264).

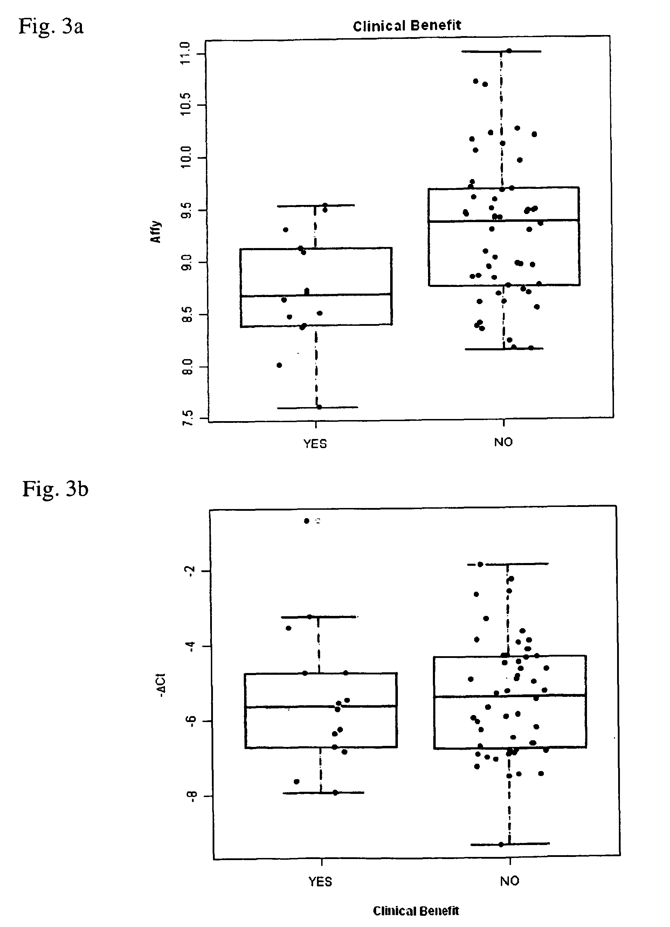

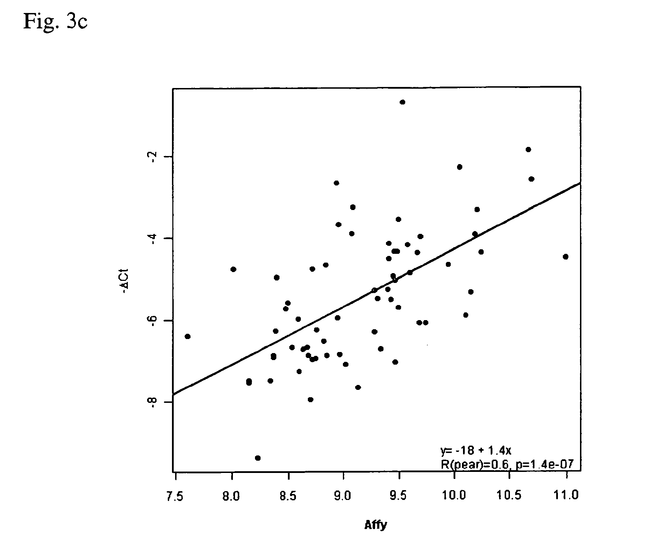

Figure 3 shows relative mRNA levels for PTP4A1 in individual patients, as assessed by Affymetrix Genechip® profiling and qRT-PCR. Figure 3a shows expression levels versus clinical outcome for Genechip® profiling and Figure 3b shows expression levels to qRT-PCR.

There was a good correlation between Genechip® and qRT-PCR measurements of the PTP4A1 mRNA transcript (Figure 3c; pearson's p=0.6, p<0.01). As observed with Genechip® profiling, PTP4A1 mRNA levels assessed using qRT-PCR appeared to correlate with response to erlotinib, with higher levels being observed in responders compared with non-responders.

### Discussion

By analyzing tissue samples with high-density oligonucleotide microarray technology, and applying statistical modeling to the data, we have been able to identify genes whose expression levels may be predictive of patients deriving a clinical benefit from treatment with erlotinib.

A composite criterion (defined above) was applied. It resulted in PTP4A1 as predictive marker for EGFR inhibitor treatment.

PTP4A1 is the second member of the protein tyrosine phosphatase family. It is located on chromosome 6q12 and encodes a protein belonging to a small class of prenylated protein tyrosine phosphatases (PTPs), which contains a PTP domain and a characteristic C-terminal prenylation motif. This tyrosine phosphatase is a nuclear protein, but may be primarily associated with the plasma membrane.

Several reports have associated high expression of PTP4A1 with oncogenic activity, specifically enhanced proliferation rate and cell motility and invasiveness. A recent study in non small cell lung cancer has demonstrated a functional role of PTP4A1 in the processes of cell migration and invasion.

In this study, PTP4A1 was found to be down regulated in patients deriving clinical benefit from treatment with erlotinib. The oncogenic function of this gene is a clear argument in favor of its utility as a tumour marker.

## Claims

1. An in vitro method of predicting the response of a NSCLC patient to treatment with erlotinib comprising:
determining an expression level of a PTP4A1 gene in a tumour sample of a patient and comparing the expression level of the PTP4A1 gene to a value representative of an expression level of the PTP4A1 gene in tumours of a population of patients deriving no clinical benefit from the treatment, wherein a lower expression level of the PTP4A1 gene in the tumour sample of the patient is indicative for a patient who will derive clinical benefit from the treatment.

2. The method of claim 1, wherein the expression level is determined by microarray technology.

3. The method of claim 1 or 2, wherein the PTP4A1 gene shows between 1.2 and 1.8 or more fold lower expression level in the tumour sample of the patient compared to the value representative of an expression level of the PTP4A1 gene in tumours of a population of patients deriving no clinical benefit from the treatment.

4. Use of a PTP4A1 gene for predicting the response of a NSCLC patient to EGFR inhibitor treatment, whereby the EGFR inhibitor is erlotinib.

## Patentansprüche

1. In vitro-Verfahren zur Prognose des Ansprechens eines NSCLC-Patienten auf die Behandlung mit Erlotinib, wobei das Verfahren umfasst:
das Bestimmen des Expressionsspiegels eines PTP4A1-Gens in einer Tumorprobe eines Patienten und das Vergleichen des Expressionsspiegels des PTP4A1-Gens mit einem Wert, der repräsentativ ist für einen Expressionsspiegel des PTP4A1-Gens in Tumoren einer Patientengruppe, die keinen klinischen Nutzen aus der Behandlung zieht, wobei ein niedrigerer Expressionsspiegel des PTP4A1-Gens in der Tumorprobe des Patienten einen Patienten anzeigt, der einen klinischen Nutzen aus der Behandlung zieht.

2. Verfahren nach Anspruch 1, wobei der Expressionsspiegel durch Mikroarray-Technik bestimmt wird.

3. Verfahren nach Anspruch 1 oder 2, wobei das PTP4A1-Gen einen zwischen 1.2 und 1.8 oder mehrfach niedrigeren Expressionsspiegel in der Tumorprobe des Patienten verglichen mit dem Wert zeigt, der repräsentativ ist für einen Expressionsspiegel des PTP4A1-Gens in Tumoren einer Patientengruppe, die keinen klinischen Nutzen aus der Behandlung zieht.

4. Verwendung eines PTP4A1-Gens zur Prognose des Ansprechens eines NSCLC-Patienten auf die Behandlung mit einem EGFR-Inhibitor, wobei der EGFR-Inhibitor Erlotinib ist.

## Revendications

1. *Procédé in vitro* de prédiction de la réponse d'un patient souffrant de cancer bronchique non à petites cellules (CBNPC) au traitement avec l'erlotinib, comprenant:
la détermination du niveau d'expression d'un gène de PTP4A1 dans un échantillon de tumeur d'un patient et la comparaison du niveau d'expression du gène de PTP4A1 à une valeur représentative du niveau d'expression du gène de PTP4A1 dans les tumeurs d'une population de patients ne retirant pas de bénéfice clinique du traitement, un niveau d'expression inférieur du gène de PTP4A1 dans l'échantillon de tumeur du patient indiquant un patient qui retirera un bénéfice clinique du traitement.

2. Procédé selon la revendication 1, dans lequel le niveau d'expression est déterminé par une technologie de puce à ADN.

3. Procédé selon la revendication 1 ou 2, dans lequel le gène de PTP4A1 présente un niveau d'expression entre 1,2 et au moins 1,8 fois plus bas dans l'échantillon de tumeur du patient par comparaison à la valeur représentative du niveau d'expression du gène de PTP4A1 dans les tumeurs d'une population de patients ne retirant pas de bénéfice clinique du traitement.

4. Utilisation d'un gène de PTP4A1 pour la prédiction de la réponse d'un patient souffrant de CBNPC à un traitement avec un inhibiteur d'EGFR, l'inhibiteur d'EGFR étant l'erlotinib.


### SEQUENCE LISTING

<110> F. Hoffmann-La Roche AG
<120> Predictive marker for EGFR inhibitor treatment
<130> 24411WO
<150> EP07114309.3
   <151> 2007-08-14
<160> 1
<170> PatentIn version 3.4
<210> 1
   <211> 5099
   <212> DNA
   <213> Homo sapiens
<400> 1
   gatgaccgtg gttaaggcag tagaaagcag gctcgacact gagacgcggt tccagcccta     60
   gaaggattgc attttacgat tcaggcaaac ttcgagtctc ctcatgtgac atgtgtgcac    120
   tcctggaccg ttttaattat gagaaacgtg gtttgcagca tgattccttc cagtcgataa    180
   atcggaatct ctctcgctcc caccccttct taacttcagg cttcctgcat cccggagcac    240
   tcccggcagc cccttccctc ccccgccccg gggatgctcc gactcggcgc ttagccattc    300
   atcaaccggt tcacaccggc ggcggccgcc gcggagtgac gtccggaggg ggcgggcctc    360
   cgcccccgcc tgtcggctcc tggcccgcgg ttccaggccg cgattggtgg ctggagggtt    420
   gcacgtcgcg ccggctataa aggggagggc ttgtgacgca agggcgcctc ggcgcgtgta    480
   ttggctcctt cggctgcggg ccggctcggc tacgcgctct gctccgagcc gctcactgca    540
   tggtagagtc tggtgccccc gccgccgcct gcatcgccgc caccgccgct ccgccacgac    600
   caccgccgcc tcctgccctg cagccaccgc caccgcctgt gtcgccgccg cctcgggacc    660
   ggctgtatga ttaggccaca atcttcaatg agtaaacata ttcctcaatt ctgtggtgtt    720
   cttggtcaca catttatgga gtttctgaag ggcagtggag attactgcca ggcacagcac    780
   gacctctatg cagacaagtg aactgtagaa actgattact gctccaccaa gaagccccca    840
   taagagtggt tatcctggac acagaagtgt tgaattgaaa tccacagagc attttacaag    900
   agttctgacc tggatggggt aaacctcagt gcacttcttt tctgttggcc tcagtattac    960
   tggattgaag aattgctgct tcttgttagg aggttcattt cacttatcat tacttacaac   1020
   ttcatactca aagcactgag aatttcaagt ggagtatatt gaagtagact tcagtttctt   1080
   tgcatcattt ctgtattcaa tttttttaat tatttcataa ccctattgag tgttttttaa   1140
   ctaaattaac atggctcgaa tgaaccgccc agctcctgtg gaagtcacat acaagaacat   1200
   gagatttctt attacacaca atccaaccaa tgcgacctta aacaaattta tagaggaact   1260
   taagaagtat ggagttacca caatagtaag agtatgtgaa gcaacttatg acactactct   1320
   tgtggagaaa gaaggtatcc atgttcttga ttggcctttt gatgatggtg caccaccatc   1380
   caaccagatt gttgatgact ggttaagtct tgtgaaaatt aagtttcgtg aagaacctgg   1440
   ttgttgtatt gctgttcatt gcgttgcagg ccttgggaga gctccagtac ttgttgccct 1500
   agcattaatt gaaggtggaa tgaaatacga agatgcagta caattcataa gacaaaagcg 1560
   gcgtggagct tttaacagca agcaacttct gtatttggag aagtatcgtc ctaaaatgcg 1620
   gctgcgtttc aaagattcca acggtcatag aaacaactgt tgcattcaat aaaattgggg 1680
   tgcctaatgc tactggaagt ggaacttgag atagggccta atttgttata catattagcc 1740
   aacatgttgg cttagtaagt ctaatgaagc ttccatagga gtattgaaag gcagttttac 1800
   caggcctcaa gctagacaga tttggcaacc tctgtatttg ggttacagtc aacctatttg 1860
   gatacttggc aaaagattct tgctgtcagc atataaaatg tgcttgtcat ttgtatcaat 1920
   tgacctttcc ccaaatcatg cagtattgag ttatgacttg ttaaatctat tcccatgcca 1980
   gaatcttatc aatacataag aaatttagga agattaggtg ccaaaatacc cagcacaata 2040
   cttgtatatt tttagtacca tacagaagta aaatcccagg aactatgaac actagacctt 2100
   atgtggttta ttccttcaat catttcaaac attgaaagta gggcctacat ggttatttgc 2160
   ctgctcactt tatgtttaca tctcccacat tcataccaat atacgtcagg tttgcttaac 2220
   cattgatttt tttttttttt taccaagtct tacagtgatt attttacgtg tttccatgta 2280
   tctcactttg tgctgtatta aaaaaacctc cattttgaaa atctacgttg tacagaagca 2340
   catgtcttta atgtcttcag acaaaaaagc cttacattaa tttaatgttt gcactctgag 2400
   gtgcaactta acagggaggg cctgagaaaa gaatgggagg gggctattaa ttatttttag 2460
   caaaatgttg cctttgtctt gtgcaaacat gtagaatatg ctctttaatt tagtaaaata 2520
   tttttttaaa aggtagagat gctttgttat tgtaatcata aacttcctga aattcttgta 2580
   atttttttcc catacttatc agaagtgtgt ttaccaactt atttttgttt gaaagtgtga 2640
   tttttttttt ccttcccaac ctctcttgca aaaaaagaaa tgggtttctg ctaatgaatt 2700
   gagcagacat ctaatatttt atatgccttt tgagctgtgt aacttaatat ttggatactt 2760
   gacaatttgt tttattatgt aattgataaa atggtgatgt gtattaatgt tagttcaacc 2820
   atatatttat actgtctggg gatgtgtggt tatagttctg tgggagaaat aattttgtca 2880
   gtgttcacca gcttgtaaaa acttagtgcg agagctgaaa catctaaata aataatgaca 2940
   tgcatttatc atcattgaga ttggtttgct taaaattaac ttattttgta gaagacaaaa 3000
   tgaattgcac ttcacttaat gtgtgtcctc atctttttac aaataaatga aggattataa 3060
   atgatgtcag cattttagta aacttttaga caaaatttgt tagggtcatt catgaaaact 3120
   ttaatactaa aagcactttc cattatatac tttttaaagg tctagataat tttgaaccaa 3180
   tttattattg tgtactgagg agaaataatg tatagtagag gacagccttg gtttgtaaag 3240
   ctcagttcca ctagttcatg gttttgtgca acttctgagc ctcagttttc tcctttgcaa 3300
   attaataatt acataccttt atagattttg aaattaattt aaatattagt atttggtaca 3360
   tgaaggctta atgttaagtt tcctttaatg atccacaata atccctttga tcacgttaat 3420
   ctaaatctag atgtctttgt ctaatttttt ttgaatagca gttataaatg taaaggactc 3480
   aaagtttaag taaaaagtga tactccacct tgtgtttcaa agaatttagt tccacctctt   3540
   cataccagtt taacacttaa tatatttcat tggattttag acagggcaaa aggaagaaca   3600
   ggggcctctg gaggcccttg gttatttaaa tcttggatta tttgtgatag taatcacaaa   3660
   tttttggcta atttttaacc tgaggttttg tttttttttt aaaggaaatg cagcctagtc   3720
   ttgagaacat aattttatat aatcaattac taaatgttaa actattacca cacagcccat   3780
   aaaacagcat ttgcgtttat tgagagagag gatgtgccat catgattaat gaaaactatc   3840
   ttttgagttt gaaaagaaat taatttgcag tgtttggatt gtatatatgg tgctaaaaat   3900
   aaattaattt actttataaa ccttatctgt acattatacg atgtgatgaa atttgctttt   3960
   tatccaaata ttttgtatct tgtaaatatg gctaattata ggaatgccta taatacatct   4020
   tagattcctt atatctaata agagttcaaa gagttatgag ttgaagtctt gaatgcagga   4080
   aactatctga tagtgttcta aaatttggtt acttgggttt ggataccctt agtgggatga   4140
   tgtaaataga ggctagctac ctaggcttgt ctatagcaac cataatgttg atgtaagtaa   4200
   tgcggttact gaatcataag aaaatgccat ctctttttag ttgaaggaaa actctggaag   4260
   taggtgccat tggtcattct gcagtgcact gcaaccattg tttcccctag tgccctcttt   4320
   tccctagggc attgctctcc tattcccacg ccttaacaca gctctatacc tagaagcagc   4380
   cagcccaggc atgcagtcac atttaatcac atcccccttc tagagtgctt caaaatgatg   4440
   tagtccctca acttggctaa agaatctcaa tctcttgaaa tttatttttt taatgtcata   4500
   ttcatctggt aaatatctac tgtttgccag gcatttaaga atatggcaaa gaacataaaa   4560
   gatggtgtca ccagattttg gtcaccaatg agtacccgac ccgttgccat gattaagaga   4620
   gaatgctttc tattggagtt tcaggaaata taatttgaga atactttaaa gggaagtgga   4680
   agtataagtg aatgatattt ttcttttaca tgtaaacaat gaagttattt caaagttaag   4740
   ttttaaacaa aatacatgaa gtagtgtctg ccatacatgt taatattcta cattcttgct   4800
   tccttaaatt aatatgtttg tgtgtatata tgtgcctcac acctgaattg aaaattaaag   4860
   actggtttaa aagtggttta aaagtgacat ttaatgtttc tccattacgt ttggggtaac   4920
   cagcctaagt ggaatcttgg aaggaaagta agggaaaaac ttgtatttgc cttcaatgaa   4980
   ttaaaccagt gatatgtgtt aacgtatgaa tgaaaggatt gatggtgatt ttataattat   5040
   atatattgcc gcagtaacca gttaataaat tgatagctac caaaaaaaaa aaaaaaaaa    5099